# EUROPEAN PATENT APPLICATION

(11) **EP 1 577 381 A1**
(43) Date of publication of application: **21.09.2005**
(21) Application number: 05101982.6
(22) Date of filing: 14.03.2005
(51) Int. Cl.: C12N 5/16, G01N 33/50, A61K 39/00

(54) **Tumor/B-cell hybrid cells and uses thereof**

(30) Priority: 15.03.2004 US 802097
(71) Applicant: Moviglia, Gustavo Antonio, Buenos Aires 1245 (AR)
(72) Inventor: Moviglia, Gustavo Antonio, Buenos Aires 1245 (AR)
(74) Representative: Holmberg, Martin Tor

(57) **Abstract**

The present invention is directed to methods and compositions for slowing or inhibiting the growth of tumors and decreasing the size of existing tumors. The compositions include dendritic cells contacted with tumor/B-cell hybrid cells and various T-cells contacted with tumor/B-cell hybrid cells (TBH cells). The present invention further encompasses methods of generating such compositions and methods of use of such compositions.

## Description

### FIELD OF THE INVENTION

The present invention is in the field of biotechnology, more particularly in the fields of immunology and cancer therapies. The present invention relates to compositions and methods of generating specific immune responses. In particular, the invention relates to methods for the use of Tumor/B-cell hybrid cells to stimulate dendritic cells or T-Cells ex vivo for use as vaccines and/or immunotherapeutics to slow or inhibit the growth of cancer and/or to reduce the malignant mass (cancer or sarcoma cell).

### BACKGROUND

The immune system is capable of killing tumor cells, including primary tumors and metastatic tumors. However, despite this ability, tumor cells are not checked by the immune system and can grow uncontrolled within the host. The lack of an immune response may be caused in part by three problems: lack of immune recognition of the tumor cells as foreign entities, anti-inflammatory secretions by the tumor cells that suppresses or leads to deterioration of the immune system, and development of tolerance by suppressor cells. Most immunotherapeutic approaches address the latter two problems. Only with recent advances in understanding of the mechanism of self-tolerance has the first problem been amenable to solution.

A number of approaches have been developed to address these problems. Early attempts included use of antigen presenting cells fused to tumor cells. The rationale was that the tumor cells often have down regulated major histocompatability complex (MHC) genes (primarily class I). Without expression of the MHC genes, the tumor cells do not present antigens for recognition by the immune system. By fusing the tumor cells to antigen presenting cells (APCs), the tumor antigens would be presented for recognition by the immune system. The tumor/APC hybrids would then be injected into the patient as a vaccine. Tumor/B-cell hybrid cells were tested in both mouse and human systems. The two studies showed extended survival times in mouse and human, respectively. (Guo, et al. 1994 and Moviglia, 1996) However, the prevailing view is that dendritic cells are better antigen presenting cells, so a number of studies have been conducted with dendritic cells fused to tumor cells. (Tanaka, et al. 2000, Gong, et al. 2002, Homma, et al. 2001, Wang, et al. 1998, Celluzzi, et al. 1998, Gong, et al. (I) 2000, Gong, et al. (III) 2000, Li, et al. 2001, Gong, et al. 1997). Such methods may suffer from the ability of tumor cells to suppress an immune response. In addition, immature dendritic cells are better at processing antigens for presentation, while mature dendritic cells are better at antigen presentation. Thus there is a need for methods and compositions that can circumvent the suppression of the immune recognition by the tumor cells and for methods and compositions that are efficient at both processing antigens and presenting antigens.

Additionally, mature dendritic cells are not ideal for such procedures given that they have a short life span in culture of approximately 15 days. The efficiency of cell fusion with mature dendritic cells is very low regardless of the method used (none produces greater than 20% efficiency). In addition, mature dendritic cells do not proliferate significantly in culture. Due to the low efficiency of fusion and the general lack of proliferation, it is necessary to start with large numbers of tumor and dendritic cells to obtain enough hybrid cells for immunization. Furthermore, it is difficult to perform multiple immunizations over time with hybrid cells from the same population. Use of non-homogenous hybrid cells, i.e., hybrid cells generated at different times may contribute to the limited efficacy of tumor/dendritic hybrid cells as vaccines observed to date.

Another method that has been tried is loading of dendritic cells *ex vivo* with an antigen. Various embodiments have been tested using different methods of loading the dendritic cell. One embodiment involved addition of a purified known tumor antigen. (See e.g., Valone, et al. 2001) Such methods require identification of each tumor antigen to be used in the therapy. A disadvantage of this method is that the antigen is usually tumor specific, so a particular antigen may not be effective to generate an immune response against a wide range of cancers. In addition, the antigen may even be tumor cell clone specific, so a particular antigen may not generate an immune response against an entire set of tumor cells in a given patient, particularly where the cancer has begun metastasizing. Another embodiment involved addition of tumor cell lysate to the dendritic cells. (See e.g., Heresy, et al. 2003) This method allows treatment of a broader range of cancer cell clone types and population, but the lysate is not processed in any way, so the efficiency of up take by the dendritic cell is lower. Additionally, tumor lysates used in such methods may suffer from a low concentration of antigen and therefore require a significant sample of tumor cells from a patient to generate sufficient numbers of lysate-loaded-dendritic cells for the entire immunization program. Thus there is a need for a method of loading *ex vivo* that can properly activate the dendritic cell to generate an immune response when reintroduced *in vivo*.

Another method that has been developed involves direct stimulation of CD8⁺ cells with dendritic cells fused to tumor cells *ex vivo.* See for example WO 01/59073, hereby incorporated by reference in its entirety. The CD8⁺ cells are intended for injection *in vivo*. Such methods have the same disadvantages as the dendritic cell vaccines as discussed above due to the use of dendritic cells fused to tumor cells.

Thus there is a need for general methods and compositions that can stimulate the immune system of a patient to recognize and reduce or even eliminate tumors that can be applied to a broad range of tumor types in different patients and tumor cell populations within a given patient. Furthermore, there is a need for methods that can use small samples of tumor that can be obtained with less invasive biopsy methods such as fine-needle aspirates where such small samples would be sufficient for the entire immunization program. Finally, there is a need for methods that can avoid the immune suppression caused by tumors and self-recognition problems in general.

### SUMMARY OF THE INVENTION

The present invention addresses the above needs by providing general methods and compositions that, by their nature, can be applied to a broad range of tumor types and tumor cell clone populations. In addition, because the dendritic cells and T-cell cells are contacted *ex vivo,* the method avoids the problems of immune suppression by the tumor, the development of autoimmune reactions, and self-recognition.

One aspect of the present invention is a composition of a plurality of isolated cells including dendritic cells in contact with TBH cells. In one embodiment, the dendritic cells, original tumor cells and B-cells were all isolated from the same individual. In another embodiment, the cells are all human cells. In still another embodiment, the TBH cells were generated by fusing tumor cells dissociated from a tumor by a non-enzymatic method, a preferred embodiment of which is mechanical dissociation. In a preferred embodiment, the dendritic cells and TBH cells are in a ratio from 10:1 to 1:10, more preferably about 4:1. In yet another embodiment, the dendritic cells were isolated from an individual by a method including a negative selection step that removed non-dendritic cell mononucleocytes. In a preferred embodiment, at least 30% of the dendritic cells are immature or precursors, at least 40% of the dendritic cells are immature or precursors, at least 50% of the dendritic cells are immature or precursors, at least 60% of the dendritic cells are immature or precursors, at least 70% of the dendritic cells are immature or precursors, at least 80% of the dendritic cells are immature or precursors, at least 90% of the dendritic cells are immature or precursors, at least 95% of the dendritic cells are immature or precursors, at least 98% of the dendritic cells are immature or precursors.

Another aspect of the present invention includes isolated dendritic cells that were in contact with TBH cells for a sufficient time to load the dendritic cells with tumor antigen in any of the embodiments described above or otherwise stimulate or activate the dendritic cells such that the dendritic cells will stimulate an immune response against the tumor in the recipient of such dendritic cells. In preferred embodiments, the dendritic cells had been contacted with the TBH cells for between 48 and 120 hours, preferably between 60 and 100 hours, more preferably between 60 and 80 hours, and most preferably approximately 72 hours.

Yet another aspect of the present invention is a composition of a plurality of isolated cells including T-Cells in contact with TBH cells. In various embodiments, the T-Cells may be selected from the following group CD3⁺CD25⁻ cells, CD3⁺CD25⁺ cells, CD3⁺CD4⁺CD25⁻ cells, CD3⁺CD4⁺CD25⁺ cells, CD3⁺CD8⁺CD25⁻ cells, and CD3⁺CD8⁺CD25⁺ cells. In one embodiment, the T-Cells, original tumor cells and B-cells were all isolated from the same individual. In another embodiment, the cells are all human cells. In still another embodiment, the TBH cells were generated by fusing tumor cells dissociated from a tumor by a non-enzymatic method, a preferred embodiment of which is mechanical dissociation. In a preferred embodiment, the T-Cells and TBH cells are in a ratio from 10:1 to 1:10, more preferably about 4:1. In yet another embodiment, the T-Cells were isolated from an individual by a method including a negative selection step that removed cells that were not the specific T-Cell of interest.

Another aspect of the present invention includes isolated T-Cells that were in contact with TBH cells for a sufficient time to simulate or otherwise activate the T-Cells in any of the embodiments described above. In preferred embodiments, the T-Cells had been contacted with the TBH cells for between 48 and 120 hours, preferably between 60 and 100 hours, more preferably between 80 and 100 hours, and most preferably approximately 96 hours.

In a preferred embodiment of the present invention relating to T-Cells, the T-Cells are CD25⁺ cells in any of the above embodiments. The isolated CD25⁺ T-Cells were in contact with TBH cells for a sufficient time to proliferate CD25⁺ T-Cells that may suppress adverse side effects in immunotherapies or may suppress the immune response to the tumor antigens. In preferred embodiments, the CD25⁺ T-Cells had been contacted with the TBH cells for between 48 and 168 hours, preferably between 60 and 120 hours, more preferably between 80 and 100 hours, and most preferably approximately 96 hours.

In a preferred embodiment of the present invention relating to T-Cells, the T-Cells are CD8⁺ cells in any of the above embodiments. The isolated CD8⁺ cells preferably have been contacted with the TBH cells for a time sufficient to activate and/or stimulate proliferation of the CD8⁺ cells that recognize tumor antigens. Such time is preferably between 36 and 120 hours, more preferably between 48 and 100 hours. The plurality of cells may further include CD4⁺ cells (T-helper cells). The CD8⁺ cells and the CD4⁺ cells are preferably at a ratio between 100:1 and 1:100, more preferably between 20:1 and 1:10, more preferably between 10:1 and 1:5.

In yet another aspect of the present invention a population of CD8⁺ cells is generated for each tumor clonal type in an individual by contacting such population with TBH cells generated from one of the tumor clonal types. The individual is then treated by introduction of the populations by any of the above embodiments.

The present invention further includes methods of generating any of the above compositions. The present invention further includes therapeutic methods involving introducing the above compositions into an individual in need of treatment.

One aspect includes use of the dendritic cell compositions or an additional step in the methods of generating such dendritic cell compositions as therapeutics. Such aspect includes introducing a therapeutically effective amount of such dendritic cells into an individual. The preferred therapeutically effective amount is between 1 × 10⁷ and 4 × 10⁹ cells, more preferably between 2 × 10⁸ and 1 × 10⁹ cells, more preferably approximately 5 × 10⁸ cells. In one embodiment, the dendritic cells were originally isolated from the individual or a cross matched donor. In another embodiment, the introduction is by intra-lymph node injection. In still another embodiment, the cells are introduced into the patient multiple times, preferably between two and six weeks, more preferably between three and four weeks. In preferred embodiment, the individual receives at least two introductions, more preferably at least three introductions, even more preferably at least six introductions.

Another aspect includes use of the T-Cell compositions or an additional step in the methods of generating such T-Cell compositions as therapeutics. Such aspect includes introducing a therapeutically effective amount of such T-Cell into an individual. The preferred therapeutically effective amount is between 1 × 10⁷ and 4 × 10⁹ cells, more preferably between 5 × 10⁷ and 1 × 10⁹ cells, more preferably approximately 5 × 10⁸ cells. In one embodiment, the T-Cells were originally isolated from the individual. In another embodiment, the introduction is by intra-tumoral injection, intra-lymph node injection, or intra-venous injection. In still another embodiment, the cells are introduced into the patient multiple times, preferably at least two weeks apart, more preferably at least three weeks apart, more preferably at least four weeks apart, or more preferably at least five weeks apart. In preferred embodiment, the individual receives at least two introductions, more preferably at least three introductions. In yet another embodiment, the T-Cells are introduced into a patient treated according to any of the above dendritic cell therapies.

Another aspect includes use of the CD25⁺ T-Cell compositions or an additional step in the methods of generating such CD25⁺ T-Cell compositions as therapeutics. Such aspect includes introducing a therapeutically effective amount of such CD25⁺ T-Cell into an individual. The preferred therapeutically effective amount is between 1 × 10⁷ and 5 × 10⁹ cells, more preferably between 5 × 10⁷ and 1 × 10⁹ cells, more preferably approximately 5 × 10⁸ cells. In one embodiment, the CD25⁺ T-Cells were originally isolated from the individual. In another embodiment, the introduction is by intra-venous injection. In still another embodiment, the cells are introduced into the patient multiple times, preferably at least twenty-four hours apart. In preferred embodiment, the individual receives at least two introductions, more preferably at least three introductions, even more preferably at least six introductions. In yet another embodiment, the CD25⁺ T-Cells are introduced into a patient treated according to any of the dendritic cell or CD8⁺ or CD3⁺ cell therapies of the present invention.

Another aspect includes use of the CD8⁺ cell compositions or an additional step in the methods of generating such CD8⁺ cell compositions as therapeutics. Such aspect includes introducing a therapeutically effective amount of such CD8⁺ cells into an individual. The preferred therapeutically effective amount is between 5 × 10⁷ and 5 × 10⁹ cells, more preferably between 2 × 10⁸ and 1 × 10⁹ cells, more preferably approximately 5 × 10⁸ cells. In one embodiment, the CD8⁺ cells were originally isolated from the individual. In another embodiment, the introduction is by intra-tumoral injection. In embodiments that include multiple populations of CD8⁺ cells as described above, the preferred introduction is by intra-tumoral injection of each population of CD8⁺ cells into the tumor from which the TBH cells that contacted said population were derived. In still another embodiment, the cells are introduced into the patient multiple times, preferably at least two weeks apart, more preferably at least three weeks apart. In preferred embodiment, the individual receives at least two introductions, more preferably at least three introductions. In yet another embodiment, the CD8⁺ cells are introduced into a patient treated according to any of the above dendritic cell therapies.

The present invention also includes various kits useful for practicing the methods and generating the compositions described above. Such kits include both stand alone kits that contain all needed equipment and reagents needed to perform a given method and kits containing replacement reagents for those reagents that are consumed in performing the method. One aspect is a kit for generating TBH cells. Such kits will include a reagent or apparatus for isolating B-cells and tumor cells, a reagent or apparatus for growing and activating B-cells, a reagent or apparatus for growing tumor cells, and/or a reagent or apparatus for fusing activated B-cells with tumor cells.

Another aspect is a kit for generating the dendritic cells contacted with TBH cells. Such kits will include a reagent or apparatus for isolating B-cells and tumor cells, a reagent or apparatus for growing and activating B-cells, a reagent or apparatus for growing tumor cells, a reagent or apparatus for fusing activated B-cells with tumor cells, a reagents or apparatus for isolating dendritic cells, and/or a reagent or apparatus for co-culturing dendritic cells with TBH cells.

Another aspect is a kit for generating the T-Cells contacted with TBH cells. Such kits will include a reagent or apparatus for isolating B-cells and tumor cells, a reagent or apparatus for growing and activating B-cells, a reagent or apparatus for growing tumor cells, a reagent or apparatus for fusing activated B-cells with tumor cells, a reagent or apparatus for isolating T-Cells, and/or a reagent or apparatus for co-culturing T-Cells with TBH cells.

Additional embodiments of the present invention may be found throughout the rest of the specification.

### BRIEF DESCRIPTION OF THE FIGURES

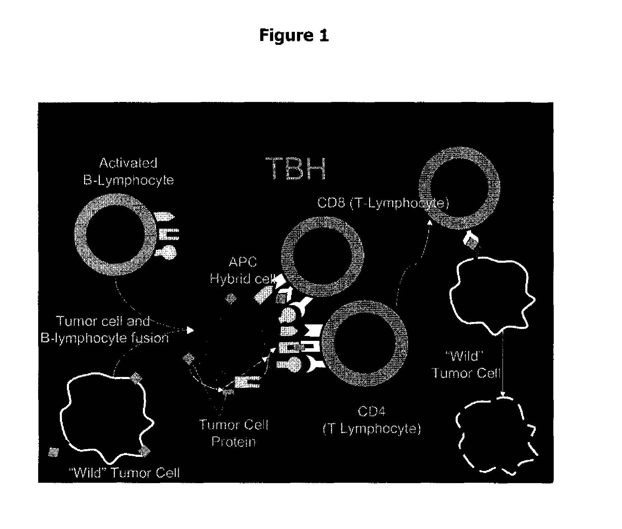

**Figure 1.** Figure 1 shows the activation of CD4+ cells and CD8+ cells either by contacting the cells with TBH cells or contacting the cells with CD4+ cells preactivated through the contact with the TBH cells.

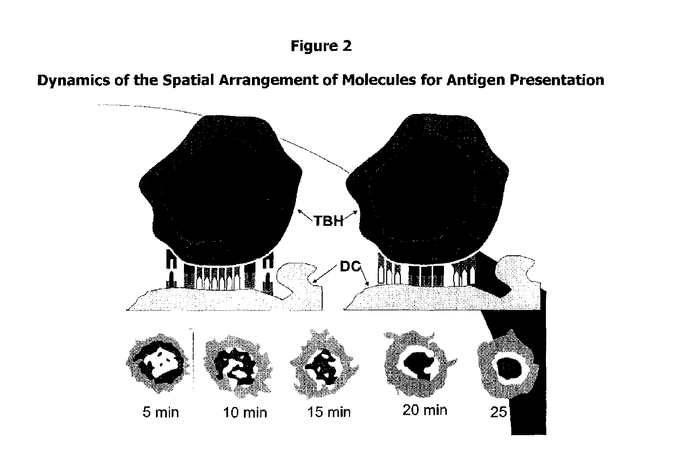

**Figure 2.** Figure 2 shows the rearrangement of MHC complexes on the surface of a TBH cell relative to the co-stimulatory complexes such as B7 and the adhesion molecules at the interface between a TBH cell and a dendritic cell (DC). The MHC complexes are shown in grey (DC side), and the co-stimulatory complexes are shown in white (DC side). The time course shows a view looking at the interface between a TBH cell presenting an antigen and a dendritic cell. Initially, the adhesion molecules are predominantly at the center of the interface, while the co-stimulatory molecules as well as MHC complexes are at the periphery. As the time course demonstrates, over time, the complexes rearrange so that the MHC complexes are at the center of the interface while the co-stimulatory complexes are at a second circle and the adhesion molecules are at the periphery. The special concentration of antigenic peptides allows a lower concentration of antigenic peptides to produce the same effect as a much higher concentration of antigenic peptides free in solution.

### BRIEF DESCRIPTION OF THE TABLES

**Table I:** Table I shows the results of administration of dendritic cells contacted with TBH cells to patients with cancer.

**Table II:** Table II shows the results of the administration of dendritic cells contacted with TBH cells followed by administration of CD8⁺ cells contacted with TBH cells to patients with cancer.

### DETAILED DESCRIPTION OF THE INVENTION

### General description of the invention and its advantages

The present invention is directed to methods and compositions for slowing or inhibiting the growth of tumors and decreasing the size of existing tumors. The compositions include dendritic cells contacted with tumor/B-cell hybrid cells and CD3⁺ cells contacted with tumor/B-cell hybrid cells (TBH cells). The present invention further encompasses methods of generating such compositions and methods of use of such compositions. In a preferred embodiment, TBH cells are generated for each tumor type in a given patient. The methods typically include generation of TBH cells though the TBH cells may already be available from prior treatments, so the generation of such TBH cells are not necessarily part of the methods of generating the compositions, but the presence of such TBH cells are useful for generating the compositions described herein. The methods of generating the activated dendritic cells may include isolation of dendritic cells, preferably from the patient to be treated, and contacting the isolated dendritic cells with the TBH cells. Once contacted, the dendritic cells may then be reintroduced into the patient. In a preferred embodiment, the reintroduction is preferably done by intra-lymph node injection.

The methods of generating the CD3⁺ cells (such CD3⁺ cells may include any or all subtypes of such cells, including without limitation, CD3⁺CD25⁻, CD3⁺CD25⁺, CD3⁺CD4⁺CD25⁻, CD3⁺CD4⁺CD25⁺, CD3⁺CD8⁺CD25⁻, CD3⁺CD8⁺CD25⁺, hereinafter "T-Cells") include isolation and/or generation of the T-Cells, preferably from the patient to be treated, and exposure of the T-Cells to TBH cells. In a preferred embodiment, the TBH cells are generated for each separate tumor in the patient. The T-Cells cells are contacted with the TBH cells. Once contacted, the T-Cells may be reintroduced into the patient by any method to any particular area or areas of the patient. In one embodiment, the reintroduction is by injection into the tumor. In another embodiment, the reintroduction is by injection into a healthy lymph node. In yet another embodiment, the reintroduction is by phlebotomy into a patient's veins. In still another embodiment, the reintroduction is by injection into the base of the tumor implant. In certain variations, the T-Cells may be reintroduced into multiple sites in the patient.

The methods of the present invention use B-cells fused to tumor cells to present tumor antigens *ex vivo* to activate dendritic cells or T-Cells. The current understanding in the art is that dendritic cells are better at antigen presentation, so most research has focused on use of dendritic cells for antigen presentation. However, the inventor of the present invention has found that the B-Cells provide advantages over dendritic cells. First, TBH cells may be more readily amplified than tumor/dendritic hybrid cells, so the methods of the present invention may be applied to smaller tumor samples. Thus a sample from a fine-needle aspirate (FNA) is sufficient for the application of the methods of the present invention, Obtaining tumor samples by FNA is less invasive than other forms of obtaining a biopsy of a tumor, so it is better for the patient.

Second, the TBH cells may be cultured for longer times. By way of comparison, TBH cells may be cultured and amplified for at least six months while tumor/dendritic hybrid cells can not be sustained longer than fifteen days and show little or no amplification over that time.

Third, TBH cells appear to be superior at generating dendritic cells that may be used to generate an immune response. While not limiting the invention to a particular theory, this may be due to the interaction between B-cells as presenting cells and dendritic cells. As shown in figure 2, the interface between TBH and dendritic cells includes MHC complexes presenting antigens, adhesion molecules, and co-stimulatory molecule complexes. Over time, these complexes arrange themselves in ordered manner where the adhesion molecules and the co-stimulatory molecule complexes form a ring around the MHC complexes at the junction between the B-cell and the dendritic cell. This sort of activation and enhanced concentration of antigen will not occur when antigen is provided to dendritic cells as individual peptides or as tumor cell lysate. Even when appropriate co-stimulatory molecules are provides, this sort of rearrangement is unlikely to occur.

While not limiting the invention to the theoretical mechanism, the TBH cells are used to load dendritic cells with antigen from the tumor. The TBH cells will process tumor antigens and present them to the dendritic cells. This form of loading the dendritic cells has distinct advantages over other methods of loading dendritic cells. Dendritic cells have been loaded by adding tumor cell lysate to dendritic cells *ex vivo*. The dendritic cells will take up proteins from the lysate for presentation; however, this is an inefficient method of preparing tumor antigens. The dendritic cells must perform their own processing of the antigenic peptides to the appropriate size. In another form, dendritic cells have been loaded with a particular antigen, in some cases with a co-stimulatory molecule. This other form of loading has the advantage that the peptide will have been tested for its ability to stimulate the immune system to recognize tumor cells. However, this form of antigen loading has the disadvantage that the immune system will only recognize the particular antigen which must be expressed in the tumor. Thus, this method requires identification and testing of each antigen to be used. The antigen may not be expressed in a wide range of different types of tumor and may not even be expressed in all tumor cell clones found in a given patient. By contrast, using TBH cells allows presentation of a wide range of possible antigens because the B-cell will provide the appropriate processing and presentation machinery to present the tumor antigens. Furthermore, since the tumor cells may be from the patient and from different tumors within the patient, the dendritic cells may be loaded with antigens from all tumor types within a given patient.

By loading the dendritic cells *ex vivo* the initiation of the immune response is begun outside of the body where there is no suppression from the tumor. *In vivo*, the tumor itself may otherwise suppress the immune response or natural self-tolerance may otherwise inhibit an immune response; however, the present invention is not intended to be limited to a particular mechanism of action.

In order for the immune system to kill cancer cells *in vivo*, the immune system must produce cytotoxic T lymphocytes (CTLs). CD8⁺ cells are the preferred CTLs. CD8⁺ cells are activated by exposure to APCs presenting antigen in the presence of appropriate co-stimulatory molecules. In a preferred embodiment, CD4⁺ helper cells are used to provide co-stimulatory molecules. In some embodiments, APCs presenting antigen alone are sufficient. Once activated by the immune system or by contacting the CTLs with an APC, including TBH cells, in the presence of CD4⁺ cells, CTLs may be amplified by exposure to the original antigen presented by another APC, including TBH cells. In some patients, a weak immune response to a tumor may have already activated a population of CD8⁺ cells that recognize antigens in the tumor. However, a preferred method is to stimulate the immune system with the dendritic cells contacted with TBH cells *ex vivo* to activate CD8⁺ *in vivo*. Then CD8⁺ cells may be isolated from the patients and amplified *ex vivo* by stimulation with the TBH cells. In another embodiment, the CD8⁺ cells are contacted with TBH cells in the presence of CD4⁺ cells. This *ex vivo* stimulation will lead to specific amplification of those activated CD8⁺ cells that recognize tumor antigens. Such *ex vivo* amplification of CD8⁺ cells has a number of advantages. First, the cancer patient may have a weakened immune system or the tumor may act to suppress the immune response. By amplification *ex vivo*, these problems are circumvented. In addition, by amplifying *ex vivo*, the CD8⁺ cells can be applied directly to the tumor by injection thus concentrating the CD8⁺ cells in the tumor for maximum effect and reducing possible killing of non-tumor cells that may share the same antigen as the tumor. Use of TBH cells as the antigen producing cells to stimulate amplification of the CD8⁺ cells has the additional advantages discussed above, specifically the easier amplification of the TBH cells. In addition, in the case where the patient was treated with dendritic cells contacted with TBH cells to activate the CD8⁺ cell, the TBH cells are more likely to generate the same antigenic fragments that initially activated the CD8⁺, so those hybrids will be the best at stimulating the proliferation of such CD8⁺ cells.

### Generation of TBHs

### Isolation of Tumor cells

The tumor cells may be isolated by any method available to one of skill in the art. Such methods include surgical removal of the tumor and taking a biopsy sample of the tumor. Numerous methods of obtaining biopsies are available to one of skill in the art including without limitation aspiration or FNA biopsy, cone biopsy, core needle biopsy, suction assisted core biopsy, endoscopic biopsy, punch biopsy, surface biopsy, and surgical biopsy (or excisional biopsy). The choice of method will be dictated by the nature and location of the tumor, the patient's other conditions, and the patient's preference. A preferred method is by fine needle aspirate (FNA), due to the less invasive nature of the procedure. The tumor cells may be allogenic, syngenic, or autologous. It is preferred that the tumor cells be autologous. It is further preferred that tumor cells be obtained from each tumor form or each clonal type of cancer cell in a patient. For example, if a primary tumor has metastasized to two other organs, then tumor cell samples would be obtained from the primary tumor and from the two other organs as well. Preferred target cancer cells for the methods of the present invention include breast cancer, stomach cancer, small intestine cancer, colon cancer, prostate cancer, lung cancer, leptomeninges cancer, glioma, melanoma, pancreatic cancer, leiomyosarcoma and blood malignancies such as Chronic Mylogenous Leukemia (CML), Hodgkins Lymphoma (HL), and Non-Hodgkins Lymphoma (NHL).

Once the tumor cell sample has been isolated, the cells need to be dissociated for fusion with B-cells. The preferred method of dissociation of the cells in the tumor sample is by mechanical cell dissociation. A more preferred method is by mechanical cell dissociation without use of proteolytic enzymes. An example would be to separate the cells using scalpel blade fragmentation. The results would then be passed through a cell dissociation sieve grinder with a metal mesh diameter of 40 micrometers (Available from Sigma Chem. Co.). Another method would be by automated disaggregation device (e.g., Medimachine available from DAKO Diagnostika GmbH, Hamburg, Germany). Yet another method of mechanical cell dissociation is by injecting cell culture medium into isolated tumor samples to release cells as described in U.S. Patent No. 5,744,363, herein incorporated by reference. The dissociated tumor cells may be expanded in culture prior to fusion. Methods for culturing tumor cells are well known in the art. See, e.g., R. Ian Freshney, Roswitha Pfragner (eds), Culture of Human Tumor Cells (2003). The times of culturing and additional factors such as growth factors will depend upon the nature of the tumor cells. By way of example, tumor cells may be cultured preferably for 48 to 240 hours. Growth factors may include, for example, insulin, preferably at 5 to 10 µg/ml, and epidermal growth factor (if the tumor is responsive to this factor), preferably at 10 to 40 µg/ml.

### Isolation of B-Cells

B-cells for use in the methods of the present invention may be isolated by any method available to one of skill in the art. One example begins with apheresis of a patient to obtain B-cells. Numerous methods of performing apheresis of are available to one of skill in the art; examples of machines that may be used include a Cobe Spectra, a Fenwal CS 3000 plus, a Vivacel Diddeco, an AS 104 Fressenius Machine, etc. Once a sample of white blood cells has been obtained, mononuclear cells may then be separated by a Ficoll-Hypaque gradient, Percoll gradient, centrifugal elutriation, etc. After this purification step, the mononuclear cells may be cultured to amplify and activate the B-cells in the mononuclear cell mixing. The mononuclear cells are preferably cultured for 72-144 hours. The media used to culture the mononuclear cells may be supplemented with factors that stimulate B-cell activation and growth. For example, the media may be supplemented with IL-4, preferably in the range of 5 to 25 µg/ml, and/or IL-6, preferably in the range of 10 to 100 µg/ml. B-cells may be obtained from the population of mononuclear cells through negative selection (for example using the StemSep for B cell kit available from Stem Cell Technology) or by positive selection (for example using CD-19 antibodies conjugated to magnetic beads).

The isolated B-cells used in the methods and compositions of the present invention are not necessarily 100% free from other cells and contaminants. In a preferred embodiment, the cells are at least 30% pure, at least 40% pure, at least 50% pure, at least 60% pure, at least 70% pure, at least 80% pure, at least 90% pure, at least 95% pure, or at least 98% pure.

### Fusion

The tumor cells and B-cells may be fused by any method available to one of skill in the art. Examples of such fusion methods include use of electricity, poly-ethylene glycol ("PEG") or Sendai virus. A preferred ratios of tumor to B-cell in the fusion reaction are from 1:10 to 10:1. A further preferred ratio is 1:4. The fused cells may then be separated if desired from the non-fused cells by a number of methods. In one embodiment, the TBH cells may be selected for positively by use of antibodies that recognize antigens on the B-cell surface such a CD-19 antibody bound to magnetic beads or a column support. Such a positive selection will select B-cells and TBH cells.

Another method is to select for fusion cells by supplementing with a reagent that selects against one or both of the unfused cells or by growing in the absence of a reagent that one or both of the unfused cells require for growth. By way of example, a tumor cell may have lost the hypoxanthine-guanine phosphoribosyl transferase. Such unfused tumor cells could be selected against by growing in HAT.

### Amplification

If desired, the TBH cells may be further amplified by growth in an appropriate media. The media may be supplemented with a molecule that will stimulate growth such as IL-2, preferably in the range of 50 to 100 IU/ml, or IL-6.

### DC exposure

### Isolation of DC cells

Dendritic cells for use in the methods of the present invention may be isolated by any method available to one of skill in the art. In order to increase the yield of dendritic cells isolated from the patient, the patient may be pre-stimulated to produce higher levels of dendritic cells. One example of a pre-stimulation method is to inject the patient daily with Granulocyte Monocyte Colony Stimulating Factor (GM-CSF), preferably in the range of 150 to 600 µg dose per injection. One example of isolation of dendritic cells from a patient's blood begins with apheresis of a patient to obtain lymphocytes. Numerous methods of performing apheresis are available to one of skill in the art; examples of machines that may be used include a Cobe Spectra, a Fenwal CS 3000 plus, a Vivacel Diddeco, an AS 104 Fressenius Machine, etc. Once a sample of white blood cells has been obtained, mononuclear cells may then be separated by a Ficoll-Hypaque gradient, Percoll gradient, centrifugal elutriation, etc. Dendritic cells may be obtained through a negative selection (for example using the StemSep for dendritic cell kit available from Stem Cell Technology).

The isolated dendritic cells used in the methods and compositions of the present invention are not necessarily 100% free from other cells and contaminants. In a preferred embodiment, the cells are at least 30% pure, at least 40% pure, at least 50% pure, at least 60% pure, at least 70% pure, at least 80% pure, at least 90% pure, at least 95% pure, or at least 98% pure. In a preferred embodiment, the dendritic cells are substantially free from CD25⁺ cells. In a preferred embodiment, the isolated dendritic cells for use in contacting the TBH cells include a reasonable fraction of immature dendritic cells or dendritic cell precursors. The dendritic cells may be at least 30% immature or precursor dendritic cells, at least 40% immature or precursor dendritic cells, at least 50% immature or precursor dendritic cells, at least 60% immature or precursor dendritic cells, at least 70% immature or precursor dendritic cells, at least 80% immature or precursor dendritic cells, at least 90% immature or precursor dendritic cells, at least 95% immature or precursor dendritic cells, or at least 98% immature or precursor dendritic cells.

### Exposure of DC cells to TBH

The dendritic cells are then exposed to the TBH cells *ex vivo* so they will be able to stimulate the immune system to recognize and kill tumor cells once reintroduced back into the patient. Dendritic cells are co-cultured with TBH cells for a sufficient time to allow the dendritic cells to generate an immune response to the cancer cells once introduced into the patient. Preferably, the cells may be cultured for between 48 and 120 hours, more preferably for approximately 72 hours. The preferred ratios of dendritic cells to TBH cells are be between 10:1 and 1:10 dendritic cells to TBH cells, more preferably, the ratio is approximately 4:1. The cells may be cultured in any media that supports the dendritic cells, such as TC199 media. The media may be enriched with factors that stimulate activation of the dendritic cells TNF α and GMCSF.

After co-culturing the cells, the dendritic cells may be separated from the TBH cells. The cells may be separated by any suitable method such as positive separation of the TBH cells using CD-19 Antibodies attached to magnetic beads.

### Treatment of patient with the DC cells

The dendritic cells after co-culturing with the TBH cells may be injected into the patient. A preferred method of administration is by injection intra-lymph node. A therapeutically effective dosage regimen should be used. A preferred therapeutically effective dosage range is from 1 x 10⁷ to 5 x 10⁹ cells, more preferably 5 x 10⁸ cells are used per injection. Preferably, patients are given one injection every two to six weeks. A preferred dosage regimen is one injection every three to four weeks. The patient may be given two such injections, but it is preferred that the patient receive at least three such injections and even more it is preferred that the patient receive at least six such injections. In addition, in a preferred embodiment, after four to eight (more preferably five to six) such injections, additional injections are delivered over longer intervals.

### T-Cell exposure

T-Cells of different types may be exposed to TBH cells for various uses. A general protocol for isolation and exposure of such T-Cells is outlined below.

### Isolation of T-Cells

T-Cells for use in the methods of the present invention may be isolated by any method available to one of skill in the art. One example begins with apheresis of a patient to obtain lymphocytes. Numerous methods of performing apheresis are available to one of skill in the art; examples of machines that may be used include a Cobe Spectra, a Fenwal CS 3000 plus, a Vivacel Diddeco, an AS 104 Fressenius Machine, etc. Once a sample of white blood cells has been obtained, mononuclear cells may then be separated by a Ficoll-Hypaque gradient, Percoll gradient, centrifugal elutriation, etc. T-Cells may be obtained from such mononuclear cells through a negative selection (for example using the appropriate StemSep kit available from Stem Cell Technology - negative selection kits are available for a variety of T-Cells) or by positive selection. The T-Cells are preferably cultured without expansion prior to exposure to the TBH cells.

The isolated T-Cells used in the methods and compositions of the present invention are not necessarily 100% free from other cells and contaminants. In a preferred embodiment, the cells are at least 30% pure, at least 40% pure, at least 50% pure, at least 60% pure, at least 70% pure, at least 80% pure, at least 90% pure, at least 95% pure, or at least 98% pure.

### Exposure of T-Cells to TBH

The purified T-Cells are co-cultured with TBH cells generated as described above. The ratios of cells and times for co-culturing are those sufficient to generate a therapeutically effective amount of the T-Cell. A preferred ratio of T-Cells to TBH cells is between 10:1 and 1:10, more preferably approximately 4:1. The cells may be co-cultured for up to 120 hours, preferably from 48 to 120, more preferably from 60 to 100 hours, even more preferably from 80 to 100 hours, and most preferably approximately 96 hours.

After co-culturing, the T-Cells may be separated from the TBH cells. A preferred method is by negative selection to remove all cells that are not the desired type of T-Cell. In addition, the TBH cells may be removed by positive selection for B-cell markers. After separation, the T-Cells may further be cultured in media. An example of such media is one enriched with IL2. A preferred time for culturing is thirty to sixty hours, and the preferred range of IL2 is from fifty to one hundred µg/ml.

### Treatment of patient with the T-Cells

The T-Cells after exposure to the TBH cells are introduced into the patient. The T-Cells are typically washed in BSS, counted, doses adjusted and administered to the patient either by intra tumoral, intra lymph node or intravenously injection, depending upon the intended use of such T-Cells. By way of example, a therapeutically effective amount of CD25⁺ T-Cells may be introduced into a patient by intravenous injection pursuant to a therapeutically effective dosage regimen. Such CD25⁺ T-Cell injections may be made in conjunction with the other immunotherapies of the present invention to lower or prevent adverse side effects that may be associated with such immunotherapies. In addition, such CD25⁺ T-Cells may be used to lower or prevent adverse side effects that may be associated with other immunotherapies.

T-Cells are introduced into the patient in therapeutically effective amounts pursuant to therapeutically effective dosage regimens. For example, CD25⁺ T-Cells should be provided in a sufficient dosage to a patient so as to suppress adverse reactions to the immunotherapies but low enough so as to not interfere with the immune response intended by the immunotherapies. A preferred dosage regimen for CD25⁺ T-Cells is single doses from 1 × 10⁷ to 5 × 10⁹ cells, which may preferably be repeated once per day for three days. In a preferred embodiment, a different set of T-Cells is generated for each tumor form in a patient by exposing the T-Cells to TBH cells from a particular tumor and then injecting the particular set of T-Cells into that tumor as therapy. A preferred therapeutically effective dose of T-Cells is 1 × 10⁷ to 1 × 10¹⁰ cells per injection. A more preferred dose is 5 × 10⁷ to 5 × 10⁹. For multiple injections, it is preferred that the injections are made at least one day apart, two days apart, four days apart, one week apart, two weeks apart, three weeks apart, four weeks apart, or five weeks apart.

The isolated T-Cells used in the methods and compositions of the present invention are not necessarily 100% free from other cells and contaminants. In a preferred embodiment, the cells are at least 30% pure, at least 40% pure, at least 50% pure, at least 60% pure, at least 70% pure, at least 80% pure, at least 90% pure, at least 95% pure, or at least 98% pure.

### CD8⁺ exposure

CD8⁺ cells are a preferred type of T-Cell of the present invention. The protocols below detail the isolating, exposure to TBH cells and general uses of such cells.

### Isolation of CD8⁺ cells

CD8⁺ cells for use in the methods of the present invention may be isolated by any method available to one of skill in the art. One example begins with apheresis of a patient to obtain lymphocytes. One of skill in the art is aware of numerous ways of performing apheresis; examples of machines that may be used include a Cobe Spectra, a Fenwal CS 3000 plus, a Vivacel Diddeco, an AS 104 Fressenius Machine, etc. Once a sample of white blood cells has been obtained, mononuclear cells may then be separated by a Ficoll-Hypaque gradient, Percoll gradient, centrifugal elutriation, etc. CD8⁺ cells may be obtained through a negative selection (for example using the StemSep for CD8 cell kit available from Stem Cell Technology) or by positive selection. The CD8⁺ cells are preferably cultured without expansion prior to exposure to the TBH cells.

The isolated CD8⁺ cells used in the methods and compositions of the present invention are not necessarily 100% free from other cells and contaminants. In a preferred embodiment, the cells are at least 30% pure, at least 40% pure, at least 50% pure, at least 60% pure, at least 70% pure, at least 80% pure, at least 90% pure, at least 95% pure, or at least 98% pure. In a preferred embodiment, the CD8⁺ cells are substantially free from CD25⁺ cells.

### Exposure of CD8⁺ cells to TBH

The purified CD8⁺ cells are co-cultured with TBH cells generated as described above. The ratios of cells and times for co-culturing are those sufficient to stimulate proliferation of CD8⁺ cells that recognize tumor cells. The preferred ratios of CD8⁺ cells to TBH are from 1:10 to 10: 1. A further preferred ratio is 4: 1. The cells may be co-cultured for up to sixty hours, preferably from thirty to sixty, even more preferably for forty-eight to sixty hours.

After co-culturing, the CD8⁺ cells may be separated from the TBH cells. A preferred method is by negative selection to removing all non-CD8⁺ cells. In addition, the TBH cells may be removed by positive selection for B-cell markers. After separation, the CD8⁺ cells may be cultured in IL2 enriched media. The preferred time for culturing is thirty to sixty hours, and the preferred range of IL2 is from fifty to one hundred µg/ml.

In another embodiment of the present invention, the CD8⁺ cells are co-cultured with TBH cells and CD4⁺ T-Cells at ratios and culture times sufficient to stimulate proliferation of CD8⁺ cells that recognize tumor cells. A preferred ratio of CD4⁺ cells to CD8⁺ cells is between 2:1 and 1:5, and more preferably between 1:1 and 1:3. The cells may preferably be co-cultured for up to sixty hours, preferably from thirty to sixty, even more preferably for forty-eight to sixty hours. The CD4⁺ cells may be removed after co-culturing by positive selection for CD4⁺ cells or negative selection for all non-CD8⁺ cells.

### Treatment of patient with the CD8⁺ cells

The CD8⁺ cells after exposure to the TBH cells are injected into the patient. The CD8⁺ cells are typically washed in BSS, counted, doses adjusted and administered to the patient either by intra tumoral, intra lymph node or intravenously injection. In a preferred embodiment, a different set of CD8⁺ cells is generated for each tumor form in a patient by exposing the CD8⁺ cells to TBH cells from a particular tumor and then injecting the particular set of CD8⁺ cells into that tumor as therapy. The CD8+ cells are introduced to the patient in a therapeutically effective dosage regimen. A preferred therapeutically effective amount of CD8⁺ cells is 5 × 10⁷ to 5 × 10⁹ cells per injection. For multiple injections, it is preferred that the injections are made at least two weeks apart, more preferably at least three weeks apart.

### Combination Therapy

It is known that CTLs must be activated by APCs in the presence of appropriate cofactors such as CD4⁺ T helper cells. The CD8⁺ cells in a given patient may already have been activated *in vivo* by recognition of the cancer cells directly. However, such pre-activation does not occur in all patients. The above embodiment directed to culturing CD8⁺ cells with TBH cells and CD4⁺ cells is one method of addressing the lack of activated CTLs. Another embodiment of the present method includes activation *in vivo* by treating the patient with the TBH exposed dendritic cells of the present invention.

Treatment with such dendritic cells will activate the CD8⁺ cells *in vivo* so that they may be expanded *ex vivo* by exposure to the TBH cells without addition of CD4⁺ cells or obtain a larger number of pre-committed CD8⁺ cells.

### Kits

The present invention also includes various kits useful for practicing methods and generating the compositions described above. Such kits include both stand alone kits that contain all needed equipment and reagents needed to perform a given method and kits containing the reagents that are consumed in performing the method. One aspect is a kit for generating TBH cells. Such kits may include a reagent or apparatus for fusing cells, a reagent or apparatus for isolating B-cells and/or tumor cells, and/or a reagent or apparatus for culturing tumor cells, B-cells, and/or TBH cells.

Another aspect is a kit for generating the dendritic cells contacted with TBH cells. Such kits may include a reagent or apparatus for fusing cells, a reagent or apparatus for isolating B-cells, tumor cells, and/or dendritic cells, a reagent or apparatus for culturing tumor cells, B-cells, dendritic cells, and/or TBH cells, and/or a reagent or apparatus for co-culturing TBH cells and dendritic cells.

Another aspect is a kit for generating the T-Cells contacted with TBH cells. Such kits may include a reagent or apparatus for fusing cells, a reagent or apparatus for isolating B-cells, tumor cells, and/or T-Cells, a reagent or apparatus for culturing the B-cells, tumor cells, TBH cells, and/or T-Cells, and/or a reagent or apparatus for co-culturing TBH cells and T-Cells.

Examples of reagents for fusing cells include, without limitation, poly-ethylene glycol or Sendai virus in appropriate concentrations and aliquots. An example of an apparatus for fusing cells include an electroporation device for fusing cells by electricity.

Examples of reagents for isolating B-Cells include conjugated antibodies for positive selection such as anti-CD19 antibodies and pluralities of conjugated antibodies for negative selection such as anti-CD2, anti-CD3, anti-CD14, anti-CD16, anti-CD56, anti-Glycophorin A antibody cocktails, where the antibodies are conjugated to an appropriate support. Examples of reagents for isolating dendritic cells include pluralities of conjugated antibodies for negative selection such as anti-CD3, anti-CD14, anti-CD16, anti-CD19, anti-CD56, anti-CD66b, and anti-Glycophorin A antibody cocktails, where the antibodies are conjugated to an appropriate support. Examples of reagents for isolating CD8⁺-Cells include conjugated antibodies for positive selection such as anti-CD8 antibodies and pluralities of conjugated antibodies for negative selection such as anti-CD4, anti-CD14, anti-CD16, anti-CD19, anti-CD56, and anti-Glycophorin A antibody cocktails, where the antibodies are conjugated to an appropriate support. An example of an apparatus for isolating such cells includes an apheresis machine.

### EXAM PLES

### Example 1: In-Vitro Production And Expansion Of Autologous Tumor - B Cell Hybrids (TBH).

**First:** the patient's Mononuclear Cells (MNC's) were collected by aphaeresis after two circulations of total blood volume through a Cobe Spectra Aphaeresis Machine.

**Second:** the collected MNC's were washed three times in a balanced salt solution (BSS). Then they were seeded on a Ficoll-Hypaque gradient (density 1.077). The precipitate is discarded and the cell ring on the ficoll phase was concentrated and washed in BSS. It gave an approximated yield of 5-10 × 10⁹ highly purified MNC's.

**Third:** MNCs were cultured and activated for 48 hrs in a TC199 media enriched with IL4 and IL6. (Moviglia, Transfusion Sci, 1996, 17(4): 643-649).

**Fourth:** After MNC's were incubated with a cocktail of monoclonal antibodies (MABs). The MABs were attached to immune magnetic beads in order to perform a negative selection for B cells (Stemsep Kit for B, Stem Cell Technology). The manufacturer's procedure was followed and 0.5-1 × 10⁹ B cells were obtained with 92% purity.

**Fifth:** The tumor specimen was extracted from the patient during surgery or from biopsy by FNA. The tissue was dissociated in a single cell suspension using mechanical techniques only. The tumor cell suspension was purified from normal tissue, stroma and white blood cells. The tumor cells were cultured for at least 48 hours in a TC199 media enriched with insulin and epidermal growth factor.

**Sixth:** The tumor cells and the activated B cells were mixed in a ratio 1:4 and suspended in a solution of poly ethylene glycol (PEG 1000 at 50% V/V in PBS) and gently pipetted for 90 to 180 seconds to induce cell fusion.

**Seventh:** The cell suspension was rapidly diluted by the addition of 10 to 50 times the original liquid volume of BSS. Then the suspension was concentrated and washed at least three times at 300 G for 5 minutes each wash at room temperature.

**Eighth:** Fused cells were cultured for three days in TC199 media enriched with IL 2. After three days of culture, a positive selection was performed using anti-CD19 antibodies attached to magnetic beads. The selected cells were again cultured for three days in TC199 media enriched with IL2. Then the cells were washed to separate the cells from the magnetic beads. Finally, purified TBH cells were cultured in TC 199 plus IL2.

### Example 2: In-Vitro Production, Expansion And Boosting Of Autologous Dendritic Cells (DC).

**First:** the patient was pre-stimulated for five consecutive days with a subcutaneous injection of 150 µg of Human Bacterial Recombinant Granulocyte Monocyte Colony Stimulating Factor (GMCSF) administered at 7 PM each day.

Second: the patient's Mononuclear Cells (MNC's) were collected by aphaeresis after two circulations of total blood volume through a Cobe Spectra Aphaeresis Machine.

Third: the collected MNC's were washed three times in a balanced salt solution (BSS). Then they were seeded on a Ficoll-Hypaque gradient (density 1.077). The precipitate was discarded and the cell ring on the ficoll phase was concentrated and washed in BSS. It gave an approximated yield of 5-10 × 10⁹ highly purified MNC's.

Fourth: MNC's were incubated with a cocktail of monoclonal antibodies (MABs). The MABs included antibodies for that bind to antigens found on MNCs other than DCs. The MABs were attached to magnetic beads in order to perform a negative selection for DC (Stemsep Kit for DC, Stem Cell Technology). The manufacturer's procedure was followed and 0.5-1.5 × 10⁹ DC were obtained with 40 to 60 % purity.

Fifth: purified DC were co-cultured with autologous TBH cells generated according to Example 1 for 72 hrs in a TC199 media at a 10:1 ratio (DC : TBH) enriched with TNF α 20 ng/ml and GMCSF 100 ng/ml. (Moviglia, Transfusion Sci, 1996, 17(4): 643-649). The TBH cells were used as tumor antigen cells.

Sixth: After the 72 hours of co-culturing, a second negative selection is performed to eliminate the TBH cells.

Seventh: DC Cell were washed in BSS, counted, doses adjusted and administered to patients by intra lymph node injection. Table I summarizes the results of application of this therapy to patients with varying types of cancer.

### Example 3: In-Vitro Production And Expansion Of Autologous Specific Committed CD8⁺ Cells (SCCDS).

**First:** the patient's Mononuclear Cells (MNC's) were collected by aphaeresis after two circulations of total blood volume through a Cobe Spectra Aphaeresis Machine.

**Second:** the collected MNC's were washed three times in a balanced salt solution (BSS). Then they were seeded on a Ficoll-Hypaque gradient (density 1.077). The precipitate was discarded and the cell ring on the ficoll phase gave an approximated yield of 1-10 x 10⁹ highly purified MNC's.

**Third:** MNC's were incubated with a cocktail of monoclonal antibodies (MABs). The MABs were attached to immune magnetic beads in order to perform a negative selection for CD8⁺ cells (Stemsep Kit for CDB, Stem Cell Technology). The manufacturer's procedure was followed and 1-3 x 10⁹ CD8⁺ cells were obtained with 98% purity.

**Fourth:** purified CD8⁺ cells were co-cultured with autologous TBH cells generated according to Example 1 (Tumor - B Lymphocyte Hybrids) for 48 hrs in a TC199 media at a 4:1 ratio (CD8 : TBH). (Moviglia, Transfusion Sci, 1996, 17(4): 643-649) The TBH cells were used as Antigen Presenting Cells.

**Fifth:** After 48 hours of co-culturing, a second negative selection was performed to remove the TBH cells.

**Sixth:** After the second negative selection, the CD8⁺ cells were cultured for 48 hrs in an IL2 enriched media resulting in a 10-fold expansion of the CD8⁺ cells.

**Seventh:** The CD8⁺ cells were washed in BSS, counted, doses adjusted and administered to patients either by intra tumoral, intra lymph node or intravenously injection. Table II summarizes the results of application of this therapy in combination with the dendritic cell therapy to patients.

**Table I:**

| **Treatment of Patients with Dendritic cells** | | | | | | |
|---|---|---|---|---|---|---|
| Patient # | Origin | Stage | ECOG | Pre-treat. | # DC | Response |
| 01 | Duodenum | IV | 1 | S | 6 | PR |
| 02 | Colon | IV | 1 | S, Ch, Rx | 5 | PR |
| 03 | Colon | IV | 1 | S, Ch, Rx | 2 | PD |
| 04 | Pancreas | IV | 2 | S, Ch, Rx | 2 | MR |
| 05 | Pancreas | IV | 2 | S, Ch, Rx | 2 | PR |
| 06 | Appendix | IV | 3 | S, Ch, Rx | 2 | SD |
| 07 | SCLC | IV | 2 | S, Ch | 3 | MR |
| 08 | NSCLC | IV | 1 | S, Ch | 6 | CR |
| 09 | NSCLC | IV | 0 | S, Ch | 6 | CR |
| 10 | NSCLC | IV | 3 | S,Ch,Rx | 1 | PD |
| 11 | NSCLC | IV | 2 | S, Ch, Rx | 3 | SD |
| 12 | NSCLC | IV | 1 | S, Ch, Rx | 2 | CR |
| 13 | Breast | IV | 1 | S, Ch, Rx | 6 | CR |
| 14 | Breast | IV | 1 | S, Ch, Rx | 2 | PR |
| 15 | Breast | IV | | S, Ch, Rx | | PR |
| 16 | Breast | IV | | S, Ch, Rx | | MR |
| 17 | Breast | IV | 3 | S, Ch, Rx | 3 | PD |
| 18 | Breast | IV | 1 | S, Ch, Rx | 6 | MR |
| 19 | Breast | IV | 1 | | 6 | PR |
| 20 | Breast | IV | 3 | S, Ch, Rx | 6 | PR |
| 21 | Breast | IV | 3 | S, Ch, Rx | 1 | MR |
| 22 | Breast | IV | 1 | S, Ch, Rx | 3 | MR |
| 23 | Ovary | IV | 2 | S | 3 | PR |
| 24 | Ovary | IV | 3 | S, Ch | 2 | SD |
| 25 | Ovary | IV | 3 | S, Ch | 1 | PD |
| 26 | Ovary | IV | 3 | S, Ch, Rx | 2 | PR |
| 27 | Prostate | IV | 1 | H | 6 | CR |
| 28 | Prostate | | 1 | H, S | 3 | SD |
| 29 | Prostate | IV | 2 | H, S | 3 | MR |
| 30 | Prostate | IV | 2 | S, H, Rx | 3 | PD |
| 31 | Bladder | IV | 1 | S, Ch | 3 | CR |
| 32 | Bladder | IV | 3 | S, Ch, Rx | 2 | PD |
| 33 | Bladder | IV | 3 | S, Ch, Rx | 2 | PD |
| 34 | Kidney | IV | 3 | S, Ch | 1 | PD |
| 35 | Kidney | IV | 2 | S, Ch | 2 | PR |
| 36 | AML | | 3 | Ch | 2 | CR |

The columns in the table correspond to the following: Origin refers to the source of the tumor cells; Stage refers to the stage of the cancer, for example, stage IV means that the cancer is inoperable or metastatic; ECOG refers to the ECOG performance status of the patient as described in Oken et al. 1982, for example, an ECOG of 3 indicates that the patient is capable of only limited self-care, confined to bed or chair more than 50% of waking hours; Pre-treat refers to the treatment that the patient has received prior to the immunotherapy where S indicates surgery, Ch indicates chemotherapy, Rx indicates radiation and H indicates hormonotherapy; # DC refers to the number of injections of dendritic cells prepared according to the above protocol that the patient has received; Response indicates the patients' response to the therapy where CR is complete remission, PR is partial remission, MR is minor response, SD is stable disease, PD is progressive disease

**Table II:**

| **Treatment of Patients with the combination therapy** | | | | | | | |
|---|---|---|---|---|---|---|---|
| Patient # | Origin | Stage | ECOG | Pre-treat. | # DC | # CD8 | Response |
| 01 | Stomach | IV | 1 | S, Ch | 6 | 1 | PR |
| 02 | Colon | IV | 0 | S, Rx | 6 | 1 | CR |
| 03 | Rectum | IV | 2 | S, Ch | 6 | 2 | PR |
| 04 | Breast | IV | 0 | S, Ch | 6 | 1 | CR |
| 05 | Breast | IV | 1 | S, Ch, Rx | 6 | 1 | CR |
| 06 | Breast | IV | 1 | S, Ch, Rx | 6 | 1 | PR |
| 07 | Breast | IV | 2 | S, Ch, Rx | 3 | 1 | PD |
| 08 | Breast | IV | 2 | S, Ch, Rx | 5 | 1 | PR |
| 09 | Breast | IV | 2 | | 2 | 1 | PR |
| 10 | Ovary | IV | 1 | S, Ch | 6 | 1 | MR |
| 11 | Ovary | IV | 3 | S, Ch | 3 | 1 | PR |
| 12 | Prostate | I | 0 | | 6 | 1 | |
| 13 | Prostate | IV | 2 | S,Ch,Rx,H | 6 | 1 | MR |
| 14 | Leiomyosarcoma | IV | 2 | S, Ch, Rx | 6 | 2 | PR |
| 15 | Leiomyosarcoma | IV | 1 | S, Ch, Rx | 6 | 3 | CR |
| 16 | Leiomyosarcoma | IV | 2 | S, Ch, Rx | 2 | 1 | PR |
| 17 | Leiomyosarcoma | IV | 3 | S, Ch, Rx | 3 | 2 | PR |
| 18 | NHL | IV | 1 | Ch | 6 | 3 | PR |

The columns in the table correspond to the following: Origin refers to the source of the tumor cells; Stage refers to the stage of the cancer, for example, stage IV means that the cancer is inoperable or metastatic; ECOG refers to the ECOG performance status of the patient as described in Oken et al. 1982, for example, an ECOG of 3 indicates that the patient is capable of only limited self-care, confined to bed or chair more than 50% of waking hours; Pre-treat refers to the treatment that the patient has received prior to the immunotherapy where S indicates surgery, Ch indicates chemotherapy, Rx indicates radiation and H indicates hormonotherapy;# DC refers to the number of injections of dendritic cells prepared according to the above protocol that the patient has received; # CD8 refers to the number of injections of CD8⁺ cells prepared according to the above protocol that the patient has received; and Response indicates the patients' response to the therapy where CR is complete remission, PR is partial remission, MR is minor response, SD is stable disease, PD is progressive disease

### REFERENCES

The following references are hereby incorporated by reference in their entirety.
1. Celluzzi, C.M., *et al*. (1998) J. Immunol. 160:3081-3085.
2. Gong, J. *et al*. (1997) Nature Medicine 3(5):1997.
3. Gong, J. (I) *et al*. (2000) Proc. Nat'l Acad. Sci. (USA) 97(6):2715-2718.
4. Gong, J. (II) *et al*. (2000) Immunology 101:316-324.
5. Gong, J. (III) *et al*. (2000) J. Immunol. 165:1705-1711.
6. Gong, J. *et al*. (2002) Blood 99(7):2512-2517.
7. Guo, Y. *et al*. (1994) Science 263:518-520.
8. Homma, S., *et al*. (2001) J. Gastroenterol. 36:764-771.
9. Li, *et al*. (2001) Cancer Immunol. Immunother. 50:456-462.
10. Hersey, P. *et al*. (2003) Cancer Immunol. Immunother. (Epub).
11. Morse, *et al*. (1999) Cancer Research 59:56-58.
12. Moviglia, G.A. (1996) Transfusion Science 17(4):643-649.
13. Oken, M.M. *et al*. (1982) Am J Clin Oncol 5:649-655.
14. Strome, S.E. *et al*. (2002) Cancer Research 62:1884-1889.
15. Tanaka, Y. *et al*. (2001) Clinical Immunology 101(2):192-200.
16. Valone, F.H. *et al*. (2001) The Cancer Journal 7(S2):S53-561.
17. Wang, J. *et al*. (1998) J. Immunol. 161(10):5516-5524.

## Claims

1. A composition comprising a plurality of cells including isolated human dendritic cells and tumor/B-cell hybrid cells wherein the tumor/B-cell hybrid cells were generated by fusing tumor cells dissociated from a tumor by mechanical dissociation with a highly enriched population of human B-cells.

2. The composition of claim 1 wherein the human dendritic cells and tumor/B-cell hybrid cells were obtained from the same individual.

3. A composition comprising isolated human dendritic cells wherein the dendritic cells have been contacted with tumor/B-cell hybrid cells for a sufficient time to load the dendritic cells with tumor antigen, wherein the tumor/B-cell hybrid cells were generated by fusing tumor cells dissociated from a tumor by mechanical dissociation with a highly enriched population of human B-cells.

4. The composition of claim 3 wherein the human dendritic cells and tumor/B-cell hybrid cells were obtained from the same individual.

5. A method of generating dendritic cells contacted with tumor/B-cell hybrid cells comprising :
a) providing isolated human dendritic cells; and
b) contacting the dendritic cells with tumor/B-cell hybrid cells for a sufficient time to load the dendritic cells with tumor antigen.

6. The method of claim 5 wherein the human dendritic cells and tumor/B-cell hybrid cells were obtained from the same individual.

7. The method of claim 5 wherein the tumor/B-cell hybrid cells were generated by fusing tumor cells dissociated from a tumor by mechanical dissociation with a highly enriched population of human B-cells.

8. The method of claim 5 comprising the additional step:
c) introducing a therapeutically effective amount of the dendritic cells into an individual in need of such cells.

9. The method of claim 8 wherein the introducing is by intra-lymph node injection.

10. A composition comprising a plurality of cells including isolated human CD8⁺ cells and tumor/B-cell hybrid cells.

11. The composition of claim 10 wherein the human CD8⁺ cells and tumor/B-cell hybrid cells were obtained from the same individual.

12. The composition of claim 10 wherein the tumor/B-cell hybrid cells were generated by fusing tumor cells dissociated from a tumor by mechanical dissociation with a highly enriched population of human B-cells.

13. The composition of claim 10 wherein the CD8⁺ cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

14. A composition comprising isolated human CD8⁺ cells wherein the CD8⁺ cells have been contacted with tumor/B-cell hybrid cells for a sufficient time to stimulate proliferation CD8⁺ cells that recognize tumor antigens.

15. The composition of claim 14 wherein the human CD8⁺ cells and tumor/B-cell hybrid cells were obtained from the same individual.

16. The composition of claim 14 wherein the CD8⁺ cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

17. The composition of claim 14 wherein the tumor/B-cell hybrid cells were generated by fusing tumor cells dissociated from a tumor by mechanical dissociation with a highly enriched population of human B-cells.

18. A method of generating CD8⁺ cells contacted with tumor/B-cell hybrid cells comprising:
a) providing isolated human CD8⁺ cells;
b) contacting the CD8⁺ cells with tumor/B-cell hybrid cells for a sufficient time to stimulate proliferation CD8⁺ cells that recognize tumor antigens.

19. The method of claim 18 wherein the human CD8⁺ cells and tumor/B-cell hybrid cells were obtained from the same individual.

20. The method of claim 18 wherein the human CD8⁺ cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

21. The method of claim 18 wherein the tumor/B-cell hybrid cells were generated by fusing tumor cells dissociated from a tumor by mechanical dissociation with a highly enriched population of human B-cells.

22. The method of claim 18 comprising the additional step:
c) introducing a therapeutically effective amount of the CD8⁺ cells into an individual in need of such cells.

23. The method of claim 22 wherein the introducing is selected from the group consisting of intratumoral injection, intra lymph node injection, intraperitoneal infusion, intrapleural infusion, intrathecal infusion, and intravenous infusion.

24. A composition comprising a plurality of cells including isolated human T-Cells and tumor/B-cell hybrid cells.

25. The composition of claim 24 wherein the human T-Cells and tumor/B-cell hybrid cells were obtained from the same individual.

26. The composition of claim 24 wherein the tumor/B-cell hybrid cells were generated by fusing tumor cells dissociated from a tumor by mechanical dissociation with a highly enriched population of human B-cells.

27. The composition of claim 24 wherein the human T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

28. A composition comprising isolated human T-Cells wherein the T-Cells have been contacted with tumor/B-cell hybrid cells for a sufficient time to stimulate proliferation human of T-Cells that recognize tumor antigens.

29. The composition of claim 28 wherein the human T-Cells and tumor/B-cell hybrid cells were obtained from the same individual.

30. The composition of claim 28 wherein the human T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

31. The composition of claim 28 wherein the tumor/B-cell hybrid cells were generated by fusing tumor cells dissociated from a tumor by mechanical dissociation with a highly enriched population of human B-cells.

32. A method of generating human T-Cells contacted with tumor/B-cell hybrid cells comprising:
a) providing isolated human T-Cells;
b) contacting the T-Cells with tumor/B-cell hybrid cells for a sufficient time to stimulate proliferation of T-Cells that recognize tumor antigens.

33. The method of claim 32 wherein the human T-Cells and tumor/B-cell hybrid cells were obtained from the same individual.

34. The method of claim 32 wherein the human T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

35. The method of claim 32 wherein the tumor/B-cell hybrid cells were generated by fusing tumor cells dissociated from a tumor by mechanical dissociation with a highly enriched population of human B-cells.

36. The method of claim 32 comprising the additional step:
c) introducing a therapeutically effective amount of the T-Cells into an individual in need of such cells.

37. The method of claim 36 wherein the introducing is selected from the group consisting of intratumoral injection, intra lymph node injection, intraperitoneal infusion, intrapleural infusion, intrathecal infusion, and intravenous infusion.

38. A composition comprising a plurality of cells including isolated human CD25⁻ T-Cells and tumor/B-cell hybrid cells.

39. The composition of claim 38 wherein the human CD25⁻ T-Cells and tumor/B-cell hybrid cells were obtained from the same individual.

40. The composition of claim 38 wherein the tumor/B-cell hybrid cells were generated by fusing tumor cells dissociated from a tumor by mechanical dissociation with a highly enriched population of human B-cells.

41. The composition of claim 38 wherein the human CD25⁻ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

42. A composition comprising isolated human CD25⁻ T-Cells wherein the CD25⁻ T-Cells have been contacted with tumor/B-cell hybrid cells for a sufficient time to stimulate proliferation of human CD25⁻ T-Cells that recognize tumor antigens.

43. The composition of claim 42 wherein the human CD25⁻ T-Cells and tumor/B-cell hybrid cells were obtained from the same individual.

44. The composition of claim 42 wherein the human CD25⁻ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

45. The composition of claim 42 wherein the tumor/B-cell hybrid cells were generated by fusing tumor cells dissociated from a tumor by mechanical dissociation with a highly enriched population of human B-cells.

46. A method of generating human CD25⁻ T-Cells contacted with tumor/B-cell hybrid cells comprising:
a) providing isolated human CD25⁻ T-Cells;
b) contacting the T-Cells with tumor/B-cell hybrid cells for a sufficient time to stimulate proliferation of T-Cells that recognize tumor antigens.

47. The method of claim 46 wherein the human CD25⁻ T-Cells and tumor/B-cell hybrid cells were obtained from the same individual.

48. The method of claim 46 wherein the human CD25⁻ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

49. The method of claim 46 wherein the tumor/B-cell hybrid cells were generated by fusing tumor cells dissociated from a tumor by mechanical dissociation with a highly enriched population of human B-cells.

50. The method of claim 46 comprising the additional step:
c) introducing a therapeutically effective amount of the CD25⁻ T-Cells into an individual in need of such cells.

51. The method of claim 50 wherein the introducing is selected from the group consisting of intratumoral injection, intra lymph node injection, intraperitoneal infusion, intrapleural infusion, intrathecal infusion, and intravenous infusion.

52. A composition comprising a plurality of cells including isolated human CD25⁺ T-Cells and tumor/B-cell hybrid cells.

53. The composition of claim 52 wherein the human CD25⁺ T-Cells and tumor/B-cell hybrid cells were obtained from the same individual.

54. The composition of claim 52 wherein the tumor/B-cell hybrid cells were generated by fusing tumor cells dissociated from a tumor by mechanical dissociation with a highly enriched population of human B-cells.

55. The composition of claim 52 wherein the human CD25⁺ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

56. A composition comprising isolated human CD25⁺ T-Cells wherein the CD25⁺ T-Cells have been contacted with tumor/B-cell hybrid cells for a sufficient time to stimulate proliferation of human CD25⁺ T-Cells that recognize tumor antigens.

57. The composition of claim 56 wherein the human CD25⁺ T-Cells and tumor/B-cell hybrid cells were obtained from the same individual.

58. The composition of claim 56 wherein the human CD25⁺ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

59. The composition of claim 56 wherein the tumor/B-cell hybrid cells were generated by fusing tumor cells dissociated from a tumor by mechanical dissociation with a highly enriched population of human B-cells.

60. A method of generating CD25⁺ T-Cells contacted with tumor/B-cell hybrid cells comprising:
a) providing isolated human CD25⁺ T-Cells;
b) contacting the T-Cells with tumor/B-cell hybrid cells for a sufficient time to stimulate proliferation of T-Cells that recognize tumor antigens.

61. The method of claim 60 wherein the human CD25⁺ T-Cells and tumor/B-cell hybrid cells were obtained from the same individual.

62. The composition of claim 60 wherein the human CD25⁺ T cells were obtained from an individual treated by providing isolated human dendritic cells, contacting the dendritic cells with tumor/B-cell hybrid cells for a sufficient time to load the dendritic cells with tumor antigen; and introducing a therapeutically effective amount of the dendritic cells into the individual.

63. The method of claim 60 wherein the tumor/B-cell hybrid cells were generated by fusing tumor cells dissociated from a tumor by mechanical dissociation with a highly enriched population of human B-cells.

64. The method of claim 60 comprising the additional step:
c) introducing a therapeutically effective amount of the CD25⁺ T-Cells into an individual in need of such cells.

65. The method of claim 64 wherein the introducing is selected from the group consisting of intratumoral injection, intra lymph node injection, intraperitoneal infusion, intrapleural infusion, intrathecal infusion, and intravenous infusion.

66. A method of slowing or inhibiting cancer growth comprising administering to an individual in need thereof an effective amount of human dendritic cells contacted in vitro with TBH cells.

67. A method of slowing or inhibiting cancer growth comprising administering to an individual in need thereof an effective amount of human CD8⁺ cells contacted in vitro with TBH cells.

68. A method of slowing or inhibiting cancer growth comprising administering to an individual in need thereof an effective amount of human T-Cells contacted in vitro with TBH cells.

69. A method of slowing or inhibiting cancer growth comprising administering to an individual in need thereof an effective amount of human CD25⁻ T-Cells contacted in vitro with TBH cells.

70. A method of lowering or inhibiting side effects of an immunotherapy comprising administering to an individual in need thereof an effective amount of human CD25⁺ cells contacted in vitro with TBH cells.

71. A method of lowering or inhibiting side effects of an immunotherapy comprising administering to an individual in need thereof an effective amount of human CD25⁺ cells contacted in vitro with TBH cells, wherein the immunotherapy is any one of the method of claims 66, 67, 68, or 69.

72. A kit for generating TBH cells comprising a reagent for fusing cells and a reagent for isolating B-cells.

73. A kit for generating TBH exposed human dendritic cells comprising a reagent for fusing cells, a reagent for isolating B-cells, and a reagent for isolating dendritic cells.

74. A kit for generating TBH exposed human CD8⁺ cells comprising a reagent for fusing cells, a reagent for isolating B-cells and a reagent for isolating CD8⁺ cells.

75. A kit for generating TBH exposed human T-Cells comprising a reagent for fusing cells, a reagent for isolating B-cells and a reagent for isolating T-Cells.

76. A kit for generating TBH exposed human CD25⁻ T-Cells comprising a reagent for fusing cells, a reagent for isolating B-cells and a reagent for isolating CD25⁻ T-Cells.

77. A kit for generating TBH exposed human CD25⁺ T-Cells comprising a reagent for fusing cells, a reagent for isolating B-cells and a reagent for isolating CD25⁺ T-Cells.
